# EUROPEAN PATENT SPECIFICATION

(11) **EP 1 907 054 B1**
(45) Date of publication and mention of the grant of the patent: **27.10.2010**
(21) Application number: 06764257.9
(22) Date of filing: 26.07.2006
(51) Int. Cl.: A61N 5/10, A61B 6/04

(54) **RADIATION THERAPY SYSTEM FOR PLANNING A RADIATION THERAPY**
STRAHLENTHERAPIESYSTEM ZUR PLANUNG EINER STRAHLENTHERAPIE
SYSTÈME DE RADIOTHÉRAPIE DE PLANIFICATION DE RADIOTHÉRAPIE

(30) Priority: 26.07.2005 DE 102005034913; 26.07.2005 US 702379 P
(43) Date of publication of application: 09.04.2008
(73) Proprietor: SIEMENS AKTIENGESELLSCHAFT, 80333 München (DE)
(72) Inventor: HERRMANN, Klaus, 90403 Nürnberg (DE); RIETZEL, Eike, 64289 Darmstadt (DE); SOMMER, Andres, 91094 Langensendelbach (DE)
(86) International application number: PCT/EP2006/064664
(87) International publication number: WO 2007/012649

(56) References cited:
- EP-A- 0 562 585
- WO-A-99/40846
- WO-A-2004/062497
- WO-A-2005/044378
- DE-A1- 3 436 444
- US-A- 5 080 100

## Description

The invention relates to a radiation therapy system having a device for obtaining image data for planning the radiation therapy, having an irradiation station having a therapeutic beam delivery device and having a patient positioning unit. The invention relates, furthermore, to methods for planning a radiation therapy of a patient.

Computer tomography units (CT units) are used in radiation therapy planning for the computer-aided generation of 3D image data of a patient's body area that contains a tumor. The image data are used to establish the irradiation fields required for a successful irradiation of the tumor, i.e. the intensity and direction of incidence of the radiation. Use is made in this case of the fact that the attenuation of the imaging X-rays can be scaled to the interaction of the therapeutic beam with the transirradiated tissue.

It is preferred to use a CT for therapy planning since; for a large volume, it provides a high resolution measurement of the attenuation of the radiation by X-ray detectors as the body is penetrated; the radiation is emitted by an X-ray source from various directions onto the patient. The X-ray source and the X-ray detectors rotate about the patient lying in a measurement opening, and so measurements that lead to the high resolution 3D image data record are carried out from a multiplicity of directions.

With state of the art CT, the patient is typically located during examination in a lying posture on a patient couch positioned in the measurement opening. In order for the anatomy to correspond during the irradiation to the anatomy used for the planning, the irradiation is also performed in this position. For irradiation purposes, use is made, on the one hand, of irradiation stations with a fixed irradiation direction and, on the other hand, of gantry-based irradiation stations in which irradiation is possible from various directions by rotating the beam of the gantry about the patient. In conventional radiation therapy, irradiation is performed with X-ray, and in the case of particle therapy use is made, for example, of protons, neutrons and carbon ions.

Depending on the irradiation station planning the directions of incidence for the therapeutic beam is restricted and, because of the underlying planning imaging, the positioning of the patient is also restricted. A change in the patient's position usually entails an internal displacement of internal organs. It is therefore to be recommended to carry out the planning imaging in the irradiation position in order to acquire the anatomy in the irradiation position as accurately as possible.

DE 101 46 915 A1 has already disclosed an X-ray apparatus, in particular a computer tomography apparatus, that is provided for examining a patient in an erect or partially erect posture and thus to the accompaniment of a natural, static loading of a specific body area. In particular, it is possible in this case to tilt the plane of rotation of the apparatus in order to align the measurement planes. DE 101 46 915 A1 discloses an imaging unit (for example C-arc X-ray apparatusor CT apparatus) that permits recordings in an erect or partially erect posture.

EP 0 220 501 B1 discloses an X-ray diagnostic system with an adjustable X-ray tube, an adjustable image recording system and a patient couch, it being possible for these system components to be moved individually in three dimensions with the aid of actuating means that can be operated by motor.

WO 99/53997 discloses a method for irradiating a patient with the aid of a horizontal particle beam by using a fixing unit to align the patient in relation to the beam in such a way that the body axis is perpendicular to the particle beam. A similar device is disclosed in US 2005/018735 A2, and in this case a robot arm with six degrees.of freedom is used to position the patient.

The system taught by EP 0 562 585 does not have a patient positioning unit and a storing unit as defined in claim 1.

It is an object of the invention to neutralize the restrictions explained above with regard to the direction of incidence in the case of radiation therapy, in particular for irradiation stations with a fixed beam direction.

This object is achieved according to the invention by means of a radiation therapy system as claimed in claim 1 and a method for planning a radiation therapy of a patient as claimed in claim 5.

In the case of the radiation therapy system according to the invention, one advantage of the device for obtaining image data resides in the fact that it is possible to use a CT gantry to examine a patient in any desired position in order to obtain image data for planning a radiation therapy and, since the radiation therapy system also has means for storing at least one "setting" parameter, which determines the alignment of the posture in space, of the patient positioning unit for planning imaging, it is rendered possible to position the patient in virtually the same posture and alignment for the irradiation.

It follows from this as a further advantage that instead of implementing the direction of incidence of the therapeutic beam in relation to the main patient axis by rotating the therapeutic beam about the patient, it is now possible for the patient to be aligned as desired, and thus, in particular, for the direction of incidence to be tuned to the anatomy even at irradiation stations with a fixed beam direction, and to be freely selected. Restrictions then still occur essentially only with regard to alignments that are unreasonable for the patient.

This advantage is rendered possible by virtue of the fact that in the alignment for the irradiation the patient is also covered for imaging during therapy planning, since otherwise the internal organs could shift in position between planning imaging and irradiation on the basis of a change in position of the patient. The tilt angles of the patient or the patient couch that form the basis of the irradiation planning are taken over into the irradiation data that characterize an irradiation operation. The patient is preferably irradiated in the alignment on which the planning was based if no relatively small correction in position takes place.

Since it is possible to dispense with gantry irradiation stations, the design of a radiation therapy system as claimed in claim 1 is simplified and yet comparable in flexibility in the case of irradiation. Above all, in the case of particle therapy systems this design leads to large savings in cost, to a flexibility in the selection of the direction of the incident beam, and thus to a good separation between tissue that is irradiated and that which is not to be irradiated.

Substantial advantages of the invention are thus, firstly, the possibility of.dispensing with the gantry and, secondly, the possibility of improving the irradiation by virtue of the fact that the irradiation planning is free of restrictions in the selection of the posture in which the irradiation is subsequently carried out.

A substantial feature of one embodiment of the invention resides in the fact that a patient is X-rayed in space in any desired body position in order to obtain image data. Here, the body position describes the alignment of the patient in space, that is to say it comprises, firstly, the posture of the patient, for example on the patient couch, and, secondly, the three-dimensional position of the patient couch (with the patient) in space. Starting from this body position, a CT gantry with a radiation source and radiation detector is moved along the main axis of the patient relative to the patient (or the patient is moved relative to the gantry) in order to obtain image data. Depending on the embodiment of the device for obtaining image data for planning a radiation therapy, it is also possible in this case, if appropriate, firstly to bring the CT gantry over the patient couch before then assuming the appropriate body position by tilting both the CT gantry and the patient couch. Alternatively, the imaging can be performed, for example, with the aid of a cone beam CT unit. The therapy planning is performed on the basis of these image data, that is to say, for example, the fixing of the direction of incidence of the therapeutic beam, and of the radiation intensity, as well as, in the case of particles, for example, the energy of the particles. Possible directions of incidence are prescribed by the irradiation device provided for use, that is to say for example, a particle beam in a horizontal direction or at 45° from above, or a gantry having angles that can be set in a plane of rotation.

Further advantageous, features and details of the invention will become evident from the description of illustrated exemplary embodiments given herein and the accompanying drawings, which are given by way of illustration only and thus are not limitative of the present invention, wherein:
- figure 1: shows a schematic of a radiation therapy sys- tem having a device for obtaining image data, and
- figure 2: shows a flowchart for illustrating the opera- tion of such a radiation therapy system.

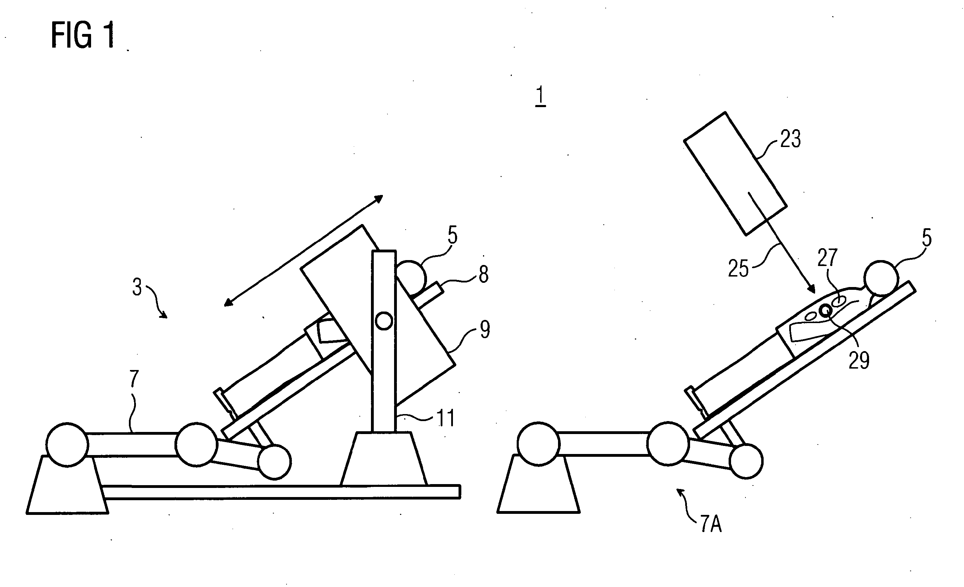

Figure 1 shows a radiation therapy system 1 having a device 3 for obtaining image data for planning a radiation therapy of a patient 5. The device 3 in this case comprises a patient positioning unit 7 that is designed in such a way that the patient 5 can be brought in space into any desired body position. A CT gantry 9 (or a cone beam CT unit) is used in this body position in order to obtain image data that is used to plan a radiation therapy of the patient 5. To this end, the CT gantry 9 is arranged in a freely moveable fashion in such a way that it is possible to carry out imaging for the purpose of radiation therapy planning in the body position of the patient 5. For example, the CT gantry 9 is held via a frame 11 in a fashion capable of rotation, displacement and tilting. A relative movement of the CT gantry or its X-ray emitters and X-radiation detectors with reference to the patient 5 can be performed, on the one hand, via the patient positioning unit through a displacement of the patient, or by a movement of the CT gantry 9.

The radiation therapy system further has an irradiation station at which the irradiation is carried out. The positioning can be carried out with the aid of the same or of a further patient positioning unit (7A in figure 1). The flexibility with regard to the alignment of the patient in his posture during the planning imaging also permits the irradiation to be carried out in a correspondingly free patient position. The patient positioning unit (7) is preferably formed by a robot that permits a free alignment and movement of the patient (5), the patient being supported, in particular, in a fashion lying on a patient couch (8) of the patient positioning unit (7) or in a fashion sitting in a patient seat of the patient positioning unit (7), or standing.

The radiation therapy system preferably further has means for carrying out radiation therapy planning (19) on the basis of the image data record. An appropriate computer-aided planning unit in the vicinity of the planning imaging and/or the irradiation station permits the radiation therapy to run in a simple way.

The radiation therapy system further has means for storing at least one setting parameter, determining the alignment of the posture in space, of the patient positioning unit (7) of the planning imaging. This renders it possible for the patient to be positioned in virtually the same posture and alignment for the irradiation. To this end, the patient positioning unit (7, 7A) reads out at least one setting parameter from the means for storage and undertakes an alignment corresponding to the setting parameter. The means for storage are preferably connected to a control system of the radiation therapy system via a data transmission system, or form a part of the control system, which is designed for driving, inter alia, patient positioning device(s), radiation source(s) and beam exit(s).

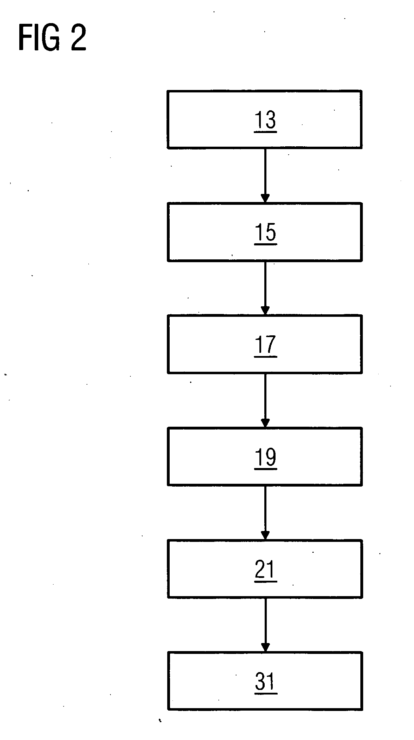

In this case, by way of example the steps illustrated in figure 2 with the aid of a flowchart are completed during operation of the radiation therapy system 1. After the prompting 13 of any desired body position by the patient 5, an imaging device (for example the CT gantry 9 in figure 1) is aligned 15 with the body position of the patient 5. Here, the body position is to be understood both as the posture of the patient 5 on, for example, a patient couch 8 or a patient seat, and the alignment of such a patient posture in space.

A movement 17 of the patient 5 relative to the imaging device 9 is used to obtain image data that, for example, permit the generation of 3D images of the patient that are suitable for therapy planning. With the aid of these data, an irradiation plan 19 is compiled that fixes the direction of incidence of the therapeutic radiation as well as further beam parameters - in a fashion designed for the body position present during the planning imaging. Subsequently, the patient 5 is positioned 21 in the irradiation position near an exit 23 of a therapeutic beam 25. Use is made to this end of, for example, an identical patient positioning unit 7A on which the patient 5 is positioned either in the same body position on which the therapeutic planning was based, or in an irradiation position that comes very near this body position. In this irradiation position, which preferably corresponds to the body position, for example organs 27 of the patient 5 are now arranged spatially in the body in relation to a tumor 29 to be irradiated in such a way that the irradiation stresses these organs 27, or body tissue to be spared, as little as possible.

A radiation therapy 31 is finally carried out in the irradiation position with the aid of the therapeutic beam 25.

Possible therapeutic beams 25 are particle beams, for example protons or carbon ions or high energy X-radiation. The beam exit 23 can be, for example, a fixed-beam beam exit, that is to say the direction of the therapeutic beam is fixed. Alternatively, the beam exit 23 can be the beam exit of a gantry with the aid of which the beam exit can be rotated about the patient in a plane.

## Claims

1. A radiation therapy system (1) for carrying out a radiation therapy of a patient, having
- a device (3) for obtaining image data for planning (19) the radiation therapy (31) of a patient (5) in a posture adopted by the patient, the device having a patient positioning unit (7) adapted to freely align in space the patient including alignment into tilted body positions, the device (3) further having an imaging device (9) capable of being aligned in a freely moveable fashion such that imaging is enabled in the posture (5) of the patient (5) that is aligned in space,
- a storing unit for storing at least one parameter of the patient positioning unit (7) during planning, said parameter determining the alignment of the posture in space, and
- an irradiation station having a therapeutic beam
delivery device (23) in front of which the patient (5) is bringable in space into the same alignment of the posture on which the planning was based with the aid of the same patient positioning unit (7) or a further patient positioning unit (7A), being adapted to read out the at least one parameter from the storing unit and to undertake an alignment corresponding to said parameter.

2. The radiation therapy system (1) as claimed in claim 1, having means for carrying out radiation therapy planning (19) on the basis of the image data record.

3. The radiation therapy system (1) as claimed in claim 1 or 2, **characterized in that** the patient positioning unit (7) comprises a robot that permits a free alignment and movement of the patient (5) being supported, in particular, lying on a patient couch (8) of the patient positioning unit (7) or sitting in a patient seat of the patient positioning unit (7), or standing.

4. The radiation therapy system (1) as claimed in one of the preceding claims, wherein the imaging device, preferably a CT gantry (9) or a cone beam CT unit, is displaceable horizontally and/or vertically, and/or along an adjustable direction, and/or tiltable about an adjustable axis such that, in particular, image planes of the imaging are arrangeable perpendicular to the arbitrarily adjustable patient alignment.

5. A method for planning a radiation therapy of a patient (5) having the following method features:
- prompting (13) of any desired body position, including tilted body positions, by the patient (5) that comes very close to a later irradiation position or corresponds to the irradiation position,
- aligning (15) an imaging device with the body position,
- prompting a relative movement (17) of the patient (5) to the imaging device in conjunction with image data of the patient (5) being simultaneously obtained by means of the imaging device, and
- planning the radiation therapy with the aid of the image data for the irradiation position.

6. The method as claimed in claim 5, wherein during prompting (13) of the body position both a posture of the patient (5) on a patient holder, in particular on a patient couch (8) or on a patient seat, is assumed, and an alignment of the patient (5) is undertaken together with the patient holder in this posture.

7. The method as claimed in claim 6, wherein said alignment of the patient (5) in this posture is undertaken via a robot unit adapted to act on the patient holder.

8. The method as claimed in one of claims 5 to 7, wherein the body position enables in the patient's body an advantageous therapeutic beam path of a therapeutic beam (25) of a radiation therapy system (1), in particular a fixed beam particle beam.

9. The method as claimed in one of claims 5 to 8, wherein during alignment of the imaging device a CT gantry (9) of the imaging device is positioned in such a way that the patient (5) is arranged on the axis of symmetry of the CT gantry (9).

10. The method as claimed in one of claims 5 to 9, wherein the patient (5) and/or imaging device are/is moved during the relative movement (17).

## Patentansprüche

1. Strahlentherapiesystem (1) zur Durchführung einer Strahlentherapie an einem Patienten, das folgendes umfasst:
- eine Vorrichtung (3) zum Gewinnen von Bilddaten für die Planung (19) der Strahlentherapie (31) bei einem Patienten (5) in einer von dem Patienten eingenommenen Körperhaltung, wobei die Vorrichtung eine Patientenpositionierungseinheit (7) umfasst, die dafür ausgelegt ist, den Patienten frei im Raum auszurichten, einschließlich der Ausrichtung in geneigten Körperpositionen, wobei die Vorrichtung (3) ferner eine Bildaufnahmevorrichtung (9) aufweist, welche in einer frei beweglichen Weise ausgerichtet werden kann, sodass eine Bildaufnahme in der Körperhaltung des Patienten (5), der im Raum ausgerichtet ist, ermöglicht wird,
- eine Speichereinheit zum Speichern mindestens eines Parameters der Patientenpositionierungseinheit (7) während der Planung, wobei der besagte Parameter die Ausrichtung der Körperhaltung im Raum bestimmt, und
- eine Bestrahlungsstation, welche eine Vorrichtung zur Bereitstellung eines Therapiestrahls (23) umfasst, vor der der Patient (5) im Raum in derselben Ausrichtung der Körperhaltung platziert werden kann, auf der die Planung basierte, mithilfe derselben Patientenpositionierungseinheit (7) oder einer weiteren Patientenpositionierungseinheit (7A), die dafür ausgelegt ist, den mindestens einen Parameter aus der Speichereinheit abzurufen und eine Ausrichtung entsprechend dem besagten Parameter vorzunehmen.

2. Strahlentherapiesystem (1) gemäß Anspruch 1, das Mittel zum Durchführen der Planung (19) einer Strahlentherapie auf der Grundlage des Bilddatensatzes aufweist.

3. Strahlentherapiesystem (1) gemäß Anspruch 1 oder 2, **dadurch gekennzeichnet, dass** die Patientenpositionierungseinheit (7) einen Roboter umfasst, der eine freie Ausrichtung und Bewegung des Patienten (5) erlaubt, der davon gehalten wird, insbesondere auf einer Patientenauflage (8) der Patientenpositionierungseinheit (7) liegend oder auf einem Patientensitz der Patientenpositionierungseinheit (7) sitzend oder in aufrechter Haltung.

4. Strahlentherapiesystem (1) gemäß einem der vorstehenden Ansprüche, wobei die Bildaufnahmevorrichtung, vorzugsweise eine CT-Gantry (9) oder eine Kegelstrahl-CT-Einheit, horizontal und/oder vertikal verschiebbar ist und/oder entlang einer einstellbaren Richtung verschiebbar ist und/oder um eine einstellbare Achse herum schwenkbar ist, sodass insbesondere Bildebenen der Bildaufnahme senkrecht zu der willkürlich einstellbaren Ausrichtung des Patienten angeordnet sind.

5. Verfahren zur Planung einer Strahlentherapie bei einem Patienten (5), das die folgenden Verfahrensmerkmale aufweist:
- Veranlassen (13) einer beliebigen gewünschten Körperposition, einschließlich geneigter Körperpositionen, des Patienten (5), die einer späteren Bestrahlungsposition angenähert ist oder der Bestrahlungsposition entspricht,
- Ausrichten (15) einer Bildaufnahmevorrichtung an der Körperposition,
- Veranlassen einer Bewegung (17) des Patienten (5) relativ zu der Bildaufnahmevorrichtung im Zusammenhang mit der gleichzeitigen Erfassung der Bilddaten des Patienten (5) mithilfe der Bildaufnahmevorrichtung, und
- Planen der Strahlentherapie mithilfe der Bilddaten für die Bestrahlungsposition.

6. Verfahren gemäß Anspruch 5, wobei während des Veranlassens (13) der Körperposition sowohl eine Körperhaltung des Patienten (5) auf einer Patientenlagerungsvorrichtung, insbesondere auf einer Patientenauflage (8) oder auf einem Patientensitz, angenommen wird, als auch eine Ausrichtung des Patienten (5) zusammen mit der Patientenlagerungsvorrichtung in dieser Körperhaltung vorgenommen wird.

7. Verfahren gemäß Anspruch 6, wobei die besagte Ausrichtung des Patienten (5) in dieser Körperhaltung mithilfe einer Robotereinheit vorgenommen wird, die dafür ausgelegt ist, auf die Patientenlagerungsvorrichtung einzuwirken.

8. Verfahren nach einem der Ansprüche 5 bis 7, wobei die Körperposition in dem Körper des Patienten einen vorteilhaften Therapiestrahlweg des Therapiestrahls (25) eines Strahlentherapiesystems (1), insbesondere eines Feststrahl-Partikelstrahls, ermöglicht.

9. Verfahren gemäß einem der Ansprüche 5 bis 8, wobei während der Ausrichtung der Bildaufnahmevorrichtung eine CT-Gantry (9) der Bildaufnahmevorrichtung derart positioniert wird, dass der Patient (5) auf der Symmetrieachse der CT-Gantry (9) angeordnet ist.

10. Verfahren gemäß einem der Ansprüche 5 bis 9, wobei der Patient (5) und/oder die Bildaufnahmevorrichtung während der relativen Bewegung (17) bewegt wird/werden.

## Revendications

1. Système de radiothérapie (1) pour exécuter une radiothérapie d'un patient, comprenant
- un dispositif (3) pour l'obtention de données d'image destinées à la planification (19) de la radiothérapie (31) d'un patient (5) dans une posture adoptée par le patient, le dispositif comprenant une unité de positionnement de patient (7) adaptée pour aligner librement le patient dans l'espace, ceci incluant l'alignement dans des positions du corps inclinées, le dispositif (3) comprenant, en outre, un dispositif d'imagerie (9) qui a la capacité d'être aligné d'une manière librement mobile de telle sorte que la formation d'images soit permise dans la posture (5) du patient (5) qui est aligné dans l'espace,
- une unité de stockage pour stocker au moins un paramètre de l'unité de positionnement de patient (7) pendant la planification, ledit paramètre déterminant l'alignement de la posture dans l'espace et
- un poste d'irradiation comprenant un dispositif de distribution de faisceau thérapeutique (23) en face duquel le patient (5) peut être amené dans l'espace dans la même posture alignée que celle sur laquelle la planification s'est basée à l'aide de la même unité de positionnement de patient (7), ou d'une autre unité de positionnement de patient (7A), qui est adaptée pour lire le au moins un paramètre dans l'unité de stockage et pour procéder à un alignement correct correspondant audit paramètre.

2. Système de radiothérapie (1) selon la revendication 1, comprenant un moyen pour exécuter une planification de radiothérapie (19) sur la base de l'enregistrement de données d'image.

3. Système de radiothérapie (1) selon la revendication 1 ou 2, **caractérisé en ce que** l'unité de positionnement de patient (7) comprend un robot qui permet un alignement et un mouvement libres du patient (5) supporté, notamment couché sur une table de patient (8) de l'unité de positionnement de patient (7) ou assis sur un siège de patient de l'unité de positionnement de patient (7) ou debout.

4. Système de radiothérapie (1) selon l'une quelconque des revendications précédentes, dans lequel le dispositif d'imagerie, de préférence un portique CT (9) ou une unité CT à faisceau conique, peut être déplacé horizontalement et/ou verticalement et/ou le long d'une direction réglable et/ou incliné autour d'un axe réglable de façon notamment à ce que les plans d'image de l'imagerie puissent être agencés perpendiculairement à l'alignement du patient pouvant faire l'objet d'un réglage arbitraire.

5. Procédé de planification d'une radiothérapie d'un patient (5) ayant les caractéristiques suivantes :
- adoption (13) d'une position du corps voulue quelconque, y compris de positions inclinées du corps, par le patient (5) qui est très proche d'une position d'irradiation ultérieure ou qui correspond à la position d'irradiation,
- alignement (15) d'un dispositif d'imagerie avec la position du corps,
- production d'un mouvement relatif (17) du patient (5) par rapport au dispositif d'imagerie en liaison avec l'obtention simultanée de données d'image du patient (5) au moyen du dispositif d'imagerie et
- planification de la radiothérapie à l'aide des données d'image relatives à la position d'irradiation.

6. Procédé selon la revendication 5, dans lequel, pendant l'adoption (13) de la position du corps, une posture du patient (5) est prise sur un support de patient, notamment sur une table de patient (8) ou sur un siège de patient, et un alignement du patient (5) dans ladite posture est réalisé dans le même temps avec le support de patient.

7. Procédé selon la revendication 6, dans lequel ledit alignement du patient (5) dans ladite posture est réalisé par l'intermédiaire d'une unité de robot adaptée pour agir sur le support de patient.

8. Procédé selon l'une quelconque des revendications 5 à 7, dans lequel la position du corps permet un trajet de faisceau thérapeutique avantageux, dans le corps du patient, d'un faisceau thérapeutique (25) d'un système de radiothérapie (1), notamment d'un faisceau de particules fixe.

9. Procédé selon l'une quelconque des revendications 5 à 8, dans lequel, pendant l'alignement du dispositif d'imagerie, un portique CT (9) du dispositif d'imagerie est positionné de telle sorte que le patient (5) est disposé sur l'axe de symétrie du portique CT (9).

10. Procédé selon l'une quelconque des revendications 5 à 9, dans lequel le patient (5) et/ou le dispositif d'imagerie sont/est déplacé/s pendant le mouvement relatif (17).
